(19) Europäisches Patentamt / European Patent Office / Office européen des brevets

(11) **EP 3 202 313 B1**

(12) # EUROPEAN PATENT SPECIFICATION

(45) Date of publication and mention
of the grant of the patent:
**27.11.2019  Bulletin 2019/48**

(51) Int Cl.:
*A61B 5/01* *(2006.01)*        *G01K 7/30* *(2006.01)*
*G01K 11/00* *(2006.01)*       *G01K 13/00* *(2006.01)*
*A61B 5/05* *(2006.01)*

(21) Application number: **15897381.8**

(22) Date of filing: **29.12.2015**

(86) International application number:
**PCT/RU2015/000953**

(87) International publication number:
**WO 2017/111651 (29.06.2017 Gazette 2017/26)**

(54) **RADIO THERMOMETER**

RADIOMETER

RADIO-THERMOMÈTRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.12.2015  RU 2015154996**

(43) Date of publication of application:
**09.08.2017  Bulletin 2017/32**

(73) Proprietor: **RTM Diagnostics, LLC
Moscow 105082 (RU)**

(72) Inventor: **VESNIN, Sergey Georgievich
Moscow 115526 (RU)**

(74) Representative: **Chimini, Francesco
Jacobacci & Partners S.p.A.
Piazza della Vittoria 11
25122 Brescia (IT)**

(56) References cited:
**RU-C1- 2 082 118        RU-C1- 2 082 118
RU-C2- 2 328 751        RU-C2- 2 328 751
US-A- 5 688 050         US-A- 5 688 050
US-B2- 8 157 442**

- **VAISBLAT A V: "Meditsinskii radiotermometr
RTM-01-RES", BIOMEDITSINSKAYA
RADIOELEKTRONIKA, IZDATEL'STVO
RADIOTEKHNIKA, RU, no. 8, 1 January 2001
(2001-01-01), pages 2-9, XP009503268, ISSN:
1560-4136**
- **FILATOV I DR A V: "Mikrovolnovyi
radiotermometr dlya izmereniya glubinnykh
temperatur biologicheskikh obektov
neinvazivnym metodom", MEASUREMENT
TECHNIQUES, CONSULTANTS BUREAU. NEW
YORK, US, no. 4, 1 January 2015 (2015-01-01),
pages 50-54, XP009502690, ISSN: 0543-1972**
- **VAISBLAT A.V.: 'Meditsinskii radiotermometr
RTM-01-RES.' BIO MEDITSINSKIE TEKHNOLOGII
I RADIOELEKTRONIKA. 2001, XP009503268**
- **A.V.FILATOV I DR.: 'Mikrovolnovyi
radiotermometr dlya izmereniya glubinnykh
temperatur biologicheskikh obektov
neinvazivnym metodom.' IZMERITELNAYA
TEKHNIKA. 2015, XP009502690**

**Description**

TECHNICAL FIELD

The invention relates to the field of medicine and medical equipment, namely, to the radiometry based on non-invasive detection of thermal abnormalities of the internal tissue of biological objects by measuring the intensity of their own electromagnetic radiation, in particular, the invention relates to the radiometer designed for non-invasive measurement of the temperature of internal tissue of a biological object.

The invention can be used in medical equipment for non-invasive measurement of the internal tissue temperature, temperature monitoring, identification of temperature variations and thermal abnormalities of internal tissues of a biological object in diagnostic complexes for early diagnosis of oncological diseases.

PRIOR ART

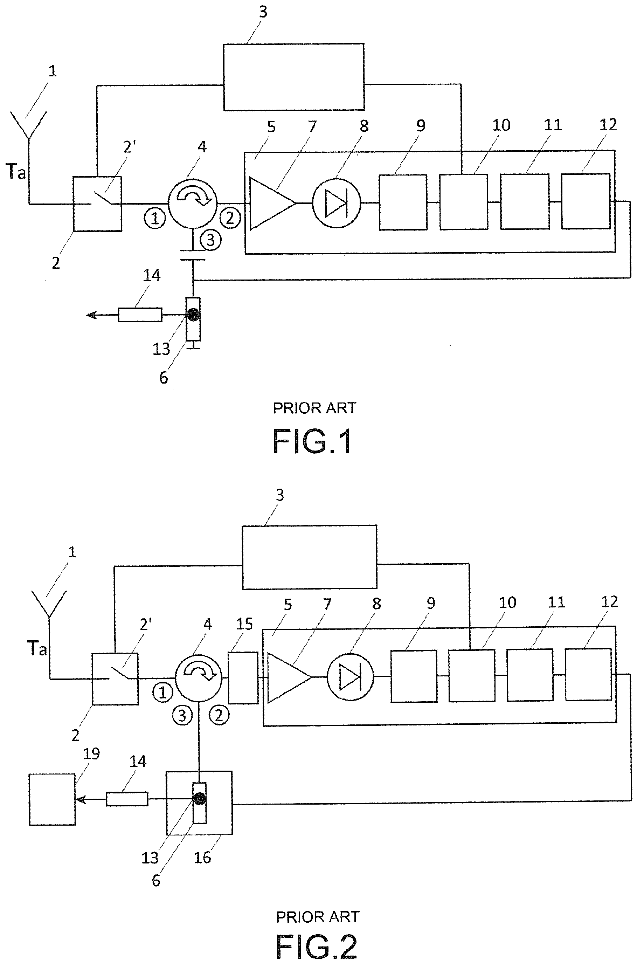

RU Pat. No.2082118 "Medical Radiometer" to A.V. Vaisblat describes the zero modulated radiometer. The block diagram corresponding to that radiometer is shown on Fig.1. The radiometer includes an aerial (1), which contacts a biological object and receives the noise signal coming from it. From the aerial outlet, the noise signal, which power is proportional to the radio brightness of the biological object's temperature, comes to a modulator (2). For the aerial, which is free of thermal losses, the temperature of noises at the aerial outlet coincides with the biological object's temperature (that is with the radio brightness temperature of the biological object); if there are losses in the aerial, then noises of the aerial itself are added to the noise temperature at the aerial outlet.

RU Pat. No. 2082118 describes the modulator which is controlled by reference voltage generator (3) and it performs the function of a switch, that is when the modulator is on, the noise signal from the aerial outlet comes to circulator (4) and then to the inlet of receiver (5), and when the modulator is off, the noise signal which power is proportional to noise temperature Tr of heated resistor (6) comes to the inlet of the receiver.

A receiver (5), consisting of a low-noise amplifier including bandpass filters (7), an amplitude detector (8), a low frequency amplifier and a selective amplifier (9), a synchronous detector (10), an integrator (11), and a direct current amplifier (12), forms voltage proportional to the difference of noise temperature Ta from the aerial outlet and noise temperature of the heated resistor. This voltage comes on a heated resistor (6), which results in thermodynamic temperature change and, consequently, changes the noise temperature of the heated resistor because in the absence of reflections, the noise temperature of heated resistor Tr coincides with its thermodynamic temperature. Due to negative feedback, voltage at the synchronous detector outlet ΔU tends toward zero

while noise temperature $T_r$ of the heated resistor tends toward noise temperature Ta from the aerial outlet.

That means that the task of measuring microwave power coming from the aerial outlet is replaced with the task of measuring temperature of the heated resistor and maintaining voltage at the synchronous detector outlet close to zero. Temperature of the heated resistor is measured with the help of a temperature sensor (13), installed on the heated resistor. To reduce the fluctuation error, the voltage coming from the temperature sensor outlet is averaged in an integrator (14) during time T, is amplified and then comes onto the indicator or for transmission to a computer.

The error of measurement of brightness temperature in radiometers depends on several factors. Firstly, due to dissipation losses in the inlet path of the radiometer, not only the noise power from the aerial outlet but thermal noise of the radiometer inlet part (circulator and selector) too come on the receiver inlet, wherein

$$\Delta T = \propto_{dis} * (T_{amb} - T_a) \ ,$$

where

$\Delta T$ is the error of measurement of the brightness temperature due to dissipation losses in the radiometer inlet path,
$T_{amb}$ is the noise temperature of the radiometer inlet part (circulator and selector),
Ta is the noise temperature from the aerial outlet,
$\propto_{dis}$ is equivalent dissipation losses of the radiometer inlet part,

therefore, the temperature of the radiometer inlet path influences on the measurement results. At a first approximation for modulated radiometers shown on Fig.1, the following equation is true

$$\propto_{dis} = \frac{\propto_{sw} - \propto_{cir}}{1 - \propto_{sw}} ,$$

where

$\propto_{cir}$ is the circulator's dissipation losses,
$\propto_{sw}$ is the modulator's dissipation losses.

To reduce the error related to temperature change of the radiometer's inlet part, dissipation losses of the circulator and modulator must be balanced. In practice, this is rather difficult to ensure because dissipation losses of microwave elements feature a certain dispersion and readjustment of dissipation losses is quite elaborated.

At that, it follows from the afore-mentioned formulas that an increase of circulator attenuation by 0.3 dB entails an error of measurement of the biological object's temperature of 1°C when the temperature of the radiometer's inlet part changes by 20°C.

Besides, medical radiometers have an error of measurement of the radio brightness temperature related to the fact that the incoming impedance of a biological object might vary within a rather wide range while the inlet resistance of the aerial is fixed. As a result, the aerial lacks ideal matching and has reflection coefficient R, so a part of the noise signal from a biological object is reflected from the aerial and does not enter the receiver. In particular, if the aerial is matched for a tissue having dielectric permeability E equal to 10, then during measurement of the temperature of a muscular tissue having E=40, the reflection coefficient will be equal to 0.33 and 10% of the signal power will be reflected from the aerial, which, correspondingly, might result in an error of brightness temperature measurement equal to 30 K.

For compensation of this error related to reflection US 4235107 A published 25.11.1980 to Ludeke describes the use schemes compensating power losses related to the end reflection coefficient by using an additional source of noise.

Similarly, in RU Pat. No. 2082118, as shown on the block diagram given on Fig.1, compensation of reflections is achieved thanks to that noise from the heated resistor (6) passes through the circulator (4) and enters the aerial. If there are reflections from the aerial, a part of the heated resistor's noise power is reflected from the aerial and goes back into the receiver, thus compensating the power reflected from the aerial. Obviously, if the noise temperature of a biological object is equal to the noise temperature entering the aerial from the heated resistor, then the reflected power will be fully compensated. But as the noise temperature of a biological object varies within a certain range it is rather difficult to fulfil this condition and the error of measurement due to signal reflection is usually quite significant. Even if the equality of noise temperatures from the aerial outlet and from the resistor is achieved, that is Ta=Tr, due to thermal losses in the circulator and modulator, the power of noise entering the aerial on the side of the heated resistor is not equal to the power of noise coming from a biological object, so full compensation does not occur.

On the block diagram shown on Fig.1, taken from RU Pat. No. 2082118, the circulator (4) is designed not only for compensation of reflections from the aerial, but also for decoupling between the receiver and aerial. Noise coming from the receiver inlet is absorbed in the circulator's balance resistor and does not enter the aerial. Decoupling of real circulators is usually not high enough to suppress fully the noise signal coming from the receiver inlet. As a result, receiver's noise that has passed through the circulator is reflected from the dead switch and enters again the receiver inlet.

The closest analogue (prototype) of the claimed invention is the radiometer described by Vaisblat A.V. in the paper "Medical Radiometer RTM-01-RES", Biomedical Technologies and Radio Electronics, No.8, 2001, P.

11-23. In the radiometer described in that article, the block diagram of which is given on Fig.2 and which contains an aerial (1) contacting a biological object, a modulator (2), which performs the function of a switch (2), a circulator (4), and a receiver (5) including a low-noise amplifier with bandpass filters (7), an amplitude detector (8), a low-frequency amplifier and a selective amplifier (9), a synchronous detector (10), an integrator (11), and a direct current amplifier (12), also a reference voltage generator (3). In the prototype radiometer, to increase decoupling between the receiver (5) and the modulator, after the circulator (4), one more nonreciprocal element - a gate (15) - is installed (see Fig.2). However, nonreciprocal elements have considerable dimensions and high price compared to other elements of a radiometer; therefore, to reduce radiometer dimensions and price, it is necessary to decerase the number of nonreciprocal elements. Besides, the prototype solution uses a Peltier element (16) to change the heated resistor temperature.

However, the design shown on Fig. 2 does not provide sufficient accuracy of measurement either because due to dissipation losses in the circulator and modulator, the power of noise entering the aerial on the side of heated resistor is not equal to the power of noise coming from a biological object so full compensation of reflections at the interface of media does not occur and the accuracy of measurement is still insufficient.

SUMMARY OF EMBODIMENT

The objective of the invention is to create a zero modulated radiometer for non-invasive detection of temperature abnormalities of internal tissues, which has a small error of brightness temperature measurement and a minimal number of nonreciprocal elements.

The solution of the said problem ensures reduction of the error of measurement of the internal temperature of a biological object and improved accuracy of the temperature radiometry method in finding malignant tumors, also decreased dimensions of the instrument, better convenience of using, and lower cost of its manufacture.

The object of the claimed invention is a radiometer for non-invasive detection of temperature abnormalities of internal tissues. The claimed radiometer contains sequentially connected:

an aerial applicator contacting a biological object,
a selector,
a circulator installed after the selector,
a receiver containing an amplifier with bandpass filters, an amplitude detector, a narrowband low-frequency amplifier and a synchronous detector, an integrator, a direct current amplifier,
a reference voltage generator connected to the selector and synchronous detector,
a Peltier element connected to the receiver outlet,
the first and second microwave loads installed on the Peltier element and thermally contacting it,
at least one temperature sensor made with a faculty of measuring the microwave temperature load, wherein
the first microwave load is made with the faculty of connection to the selector,
the selector is made with the faculty of connecting either the aerial applicator or the first microwave load to the first shoulder of the circulator,
the second shoulder of the circulator is connected to the receiver, and
the third shoulder of the circulator is connected to the second microwave load.

In an option of make, the radiometer may additionally include an attenuator installed between the outlet of the first microwave load and the selector.

The temperature sensor made with the faculty of measuring microwave load temperature may be installed on the Peltier element and/or on a microwave load.

As at least one temperature sensor, an infrared temperature sensor may be used, which is made with the faculty of remote temperature measurement, and/or a temperature sensor installed on microwave loads and/or the Peltier element and having good thermal contain with them.

The radiometer may also include an additional integrator connected to the outlet of at least one temperature sensor.

All elements of the radiometer, including the circulator, Peltier element and selector, are mounted on a heat-conductive base and have a thermal contact with it, so, the temperature of all elements of the inlet part of the radiometer (the selector, circulator, attenuator) is close to the temperature of the base they are mounted on.

So, the first side of the Peltier element is installed on the base and has a good thermal contact with it, while two microwave loads are installed on the Peltier element side that is opposite to the base, and have a good thermal contact with it.

All components of the radiometer have a common microwave signal earthing.

BRIEF DESCRIPTION OF THE DRAWINGS

Fig.1 shows a block diagram of the known from the prior art, zero modulated radiometer according to RU Pat. No. 2082118.
Fig.2 shows a block diagram of the known from the prior art, zero modulated radiometer according to the closest analogue (prototype) of the claimed invention, which has two nonreciprocal elements and in which the resistor is accommodated on a Peltier element.
Fig.3 shows a block diagram of an option of make of the zero modulated radiometer according to the claimed invention, which has a selector and two microwave loads installed on a Peltier element and connected, correspondingly, to the circulator and selector.
Fig.4 shows a block diagram of another option make of the zero modulated radiometer with a selector and two microwave loads installed on a Peltier element, wherein an attenuator is installed between the first microwave load and the selector.

DETAILED DESCRIPTION

Fig.1 shows a block diagram of the known from the prior art, zero modulated radiometer according to RU Pat.No. 2082118; at that, the block diagram is presented adapted similarly to the claimed invention, and that, known from the prior art, zero modulated radiometer consists of an aerial (1) contacting a biological object and receiving a noise signal coming from the biological object. From the aerial outlet, the microwave noise signal enters the inlet of an electronic modulator (2). In the modulator (2) of the radiometer according to RUPat. No.2082118, a switch (2') is used, which closes and opens the connection of a circulator with the aerial.

The modulator is controlled by a reference voltage generator with a clock frequency of 1 kHz. When the switch (2') of the modulator is on, the noise signal from the aerial outlet enters a circulator (4) and then enters the inlet of a receiver (5). When the switch (2') of the modulator is off, the noise signal from a heated resistor accommodated on the third shoulder of the circulator (4) is reflected from the dead switch of the modulator and enters the inlet of the circulator and then the inlet of the receiver (5).

The receiver contains a low-noise amplifier with bandpass filters (7), an amplitude detector (8), a narrowband low-frequency amplifier (9), a synchronous detector (10), an integrator (11), a direct current amplifier (12).

At the receiver outlet, voltage is formed that is proportional to the difference of the heated resistor noise temperature Tr and the temperature Ta of noise coming from the aerial outlet

$$\Delta U = k\,(T_a - T_r),$$

where

k is the amplification coefficient of the radiometer receive path,
Ta is the noise temperature from the aerial outlet,
Tr is the noise temperature of the resistor.

This signal is amplified and comes onto the heated resistor (6), resulting in a change of its thermodynamic temperature and, consequently, the resistor noise temperature Tr. Cooling of the heated resistor was achieved by way of natural air cooling.

Due to negative feedback, voltage at the synchronous detector outlet tends toward zero, and the noise temperature of the heated resistor $T_r$ tends toward the noise temperature Ta coming from the aerial outlet.

As at the synchronous detector outlet, voltage is close to zero, the noise temperature coming from the aerial outlet is equal to the noise temperature of the heated resistor.

In the absence of reflections, the noise temperature Tr of the heated resistor coincides with the thermodynamic temperature measured with the help of a temperature sensor installed on the heated resistor. To reduce the fluctuation error, voltage coming from the temperature sensor outlet is averaged in the integrator (14) and amplified.

Fig.2 shows, in a presentation form similar to the claimed invention, a block diagram of the commercial radiometer - prototype described by Vaisblat A.V. in paper "Medial Radiometer RTM-01-RES", Biomedical Technologies and Radio Electronics, No.8, 2001, P. 11-23. That radiometer consists of an aerial (1), a modulator (2), a circulator (3), a gate (15), a receiver (5), a Peltier element (16), a microwave load (6), installed on a Peltier element (16), a temperature sensor (13) measuring the temperature of microwave load (6), an integrator (14), a reference voltage generator (3) controlling the modulator (2). Wherein, in prototype radiometer same as in the analogue according to RU Pat. No. 2082118, the modulator (2) includes a switch (2'), which can only close and open the connection between the circulator and aerial.

The receiver in the prototype consists of a low-noise amplifier with bandpass filters (7), an amplitude detector (8), a narrowband low-frequency amplifier (9), and a synchronous detector (10), and integrator (11), direct current amplifier (12).

In the prototype radiometer shown on Fig.2, in contrast to the block diagram shown on Fig.1, the load temperature is controlled with the help of Peltier element (16). This allowed implementing both heating and cooling of the load. To increase decoupling between the receiver and aerial, in the prototype radiometer a second nonreciprocal element - gate (15) - is installed. This allows increasing decoupling between the switch (2') and receiver to 34 dB in the frequency spectrum of 500 mHz and reduce the level of noise coming onto the modulator (2) from the receiver (5), but enlarges outer dimensions of the device.

Thus, the prototype radiometer does not provide the required accuracy of measurement because compensation of reflections from the aerial is still insufficient, besides, the radiometer has large outer dimensions due to use of nonreciprocal elements, for example, the valve (15), which makes it inconvenient in use.

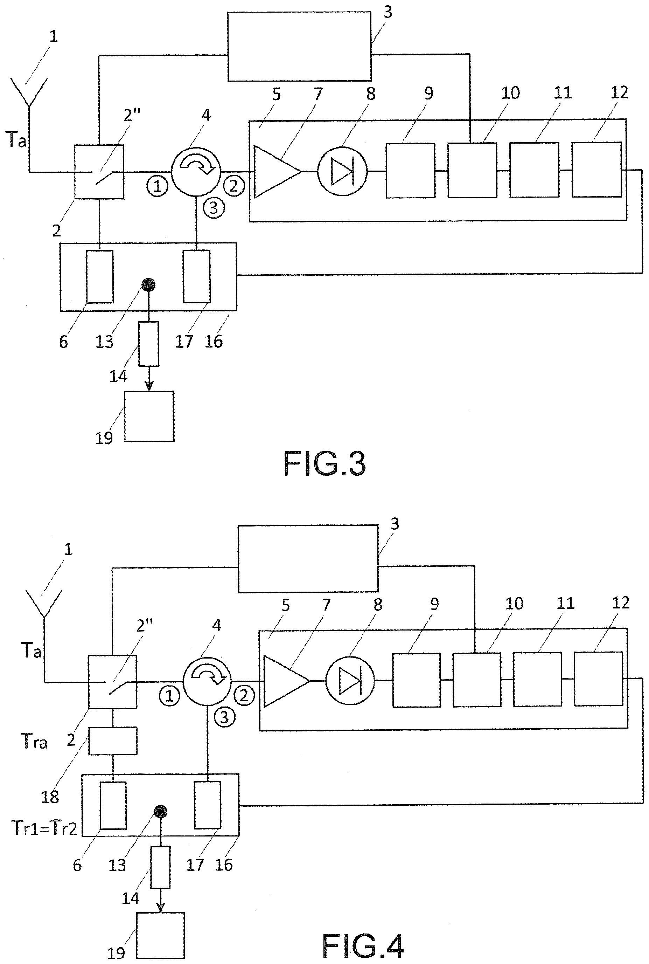

The design of the claimed radiometer is explained in detail with a reference to Figs. 3 and 4. Fig.3 shows the first option of the suggested radiometer according to the claimed invention, which additionally has a second microwave load (a second resistor) installed on the Peltier element, and the modulator has a selector (2") rather than a switch, which connects to the first shoulder of the circulator either the aerial (1) or the first load (6) (the first resistor). The selector (2") is controlled by a reference voltage generator, for example, having 1 kHz frequency. The noise signal from the outlet of selector (2") passes through a circulator (4) and enters a receiver (5).

The receiver (5) contains a low-noise amplifier with bandpass filters (7), an amplitude detector (8), a narrowband low-frequency amplifier (9), a synchronous detector (10), an integrator (11), a direct current amplifier (12).

During operation of the claimed radiometer, voltage $\Delta U$ is formed at the receiver outlet, which is proportional to the difference of the noise temperature coming from the aerial and temperature $Tr_1$ of the first heated resistor:

$$\Delta U = k\,(T_a - T_{r1}),$$

where

    k is the amplification coefficient of the first receive path of the radiometer,
    Ta is the noise temperature from the aerial outlet,
    $Tr_1$ is the noise temperature of the first microwave load (the first resistor).

This voltage is amplified and comes on the Peltier element (16). In contrast to the block diagrams of the analogous device shown in Fig.1 and Fig.2, in the claimed invention 2 microwave loads (6) and (17) are used, that is two resistors installed on the Peltier element and having good thermal contact with the Peltier element. The first load (6) is connected to the inlet of selector (2') of the modulator (2). The second load (17) is connected to the third shoulder of the circulator (4).

The temperature of loads is measured with the help of a temperature sensor (13), which may be installed on the Peltier element or at least on one of the loads and has a good thermal contact with them, then the measurement signal from the temperature sensor is integrated in an additional integrator (14) connected with the temperature sensor (13), is amplified and comes onto an indicator or into a computer (19), performing the functions of a data processing unit and a control unit.

In contrast of the block diagrams according to the analogue and prototype shown on Fig.1 and Fig.2, in the claimed technical solution, instead of the switch (2'), which is contained in the modulator and either connects the aerial outlet with the circulator or breaks the connection, a selector (2") is used, which connects either the aerial applicator or the first microwave load to the first shoulder of the circulator. In this instance, at the outlet of the receiver (5), comparison of signals coming from the aerial (1) and from the first load (6) takes place.

In the claimed invention, as the selector (2"), a SPDT-type selector (a single-pole double-trigger switch) can be used.

In some prior art solutions, attempts have also been made to use selectors in radiometer designs, for example, in the design of the radiometer according to RU Pat. 2485462 published 20.06.2013, between a modulator and a circulator, a brancher is installed, to which a two-pole selector having three inlets and two outlets is installed. In this design, three matched loads are used, wherein the first matched load is connected to a circulator, and the second and third matched loads may be commutated to the selector, and the two-pole selector in RF patent 2485462 is made with the faculty of either connecting the first outlet of the selector to a noise generator and the second outlet to the second matched load, or connecting the first outlet of the selector of the third matched load and the second outlet to the noise generator. Whereas, the modulator, brancher, circulator, selector, noise generator and source of current for it, as well as the first, second and third matched loads are installed on a thermostat plate and have an equal temperature, but there is not Peltier element in this design.

Thus, the radiometer schematic according to the claimed invention has a simpler design, contains only two matched microwave loads, which have other connections to other elements of the design. Besides, in the claimed radiometer, both loads are installed on a Peltier element, which can both heat loads and cool them, hence, loads have an equal regulated temperature that is different from the temperature of other elements of the schematic. This provides a higher accuracy of brightness temperature measurement at a minimal number of nonreciprocal elements, which reduces the outer dimensions of the design and improves convenience of its use during measurement of the internal temperature in many points of a biological object.

During radiometer operation, due to negative feedback, voltage at the synchronous detector outlet tends toward zero and noise temperature Tr1 of the first load (6) comes close to the temperature of noise $T_a$ coming from the aerial outlet.

Due to an imperfect decoupling of the circulator, a part of the receiver noise passes through the circulator (4) and enters the selector (2"). In the prototype radiometer, which block diagram is shown on Fig.2, noise was reflected from the open shoulder of switch (2') and entered the receiver inlet. In contrast to the prototype device shown on Fig.2, in the claimed invention this noise is absorbed in the first load (6) and does not get to the inlet of receiver (5). Thanks to this, in the suggested option of the radiometer, there are lower requirement to circulator decoupling and it is not necessary to install additionally a gate as it was made in the prototype (see Fig.2). This allows almost a double decrease in the sizes of the inlet microwave part of the radiometer, that is reducing the radiometer dimensions in general, which significantly simplifies radiometer manipulation during an examination, improves convenience of its use, and reduces the time required for an examination in multiple points as is the case, for example, during a breast examination.

Improvement of the measurement accuracy by compensating reflections from the inlet of aerial (1) in the suggested option of the radiometer is also achieved thanks to receipt in the aerial outlet of a noise signal from the second load (17). As it has a good thermal contact with the first load (6) through accommodation on one Peltier element, their temperatures are equal $Tr_1=Tr_2$. But since the noise temperature of the first load $Tr_1$ is close to the temperature Ta of the noise coming from the aerial outlet, then the temperature $Tr_2$ of the second load is close to the temperature of noise Ta at the aerial outlet. Thanks to this, a fuller compensation of the reflected noise power from the aerial is achieved and the accuracy of measuring the temperature of a biological object is improved.

It should be noted that due to losses in the circulator (4) and selector (2"), the power of noise coming from the side of the second load (17) onto the aerial outlet will differ from the power of noise coming from the aerial outlet, therefore, a still fuller compensation of the reflected power is provided by the radiometer option shown on Fig.4.

In the suggested radiometer option shown on Fig.4, the noise power from the outlet of the first microwave load (6) comes on an attenuator (18) connected between the outlet of the first microwave load (6) and the selector (2") and having the temperature of the inlet part of the radiometer. The noise temperature Tra at the attenuator outlet is equal to

$$Tra = Tr1 * kra + (1 - kra) * Tamb,$$

where

$k_{ra}$ is the attenuator transmission coefficient,
T amb is the noise temperature of the inlet part of the radiometer,
$Tr_1$ is the noise temperature of the first heated resistor (the first microwave load).

The radiometer functions so that the selector (2") connects to the first shoulder of the circulator either the noise signal from the outlet of aerial (1), the power of which is proportional to the temperature of internal tissues of a biological object, or the noise signal from the outlet of attenuator (18). The selector (2") is controlled by the reference voltage generator (3) having 1 kHz frequency. The noise signal from the outlet of selector (2") passes through the circulator (4) and enters the receiver (5).

The receiver, in the radiometer make option shown on Fig.4, also consists of a low-noise amplifier with bandpass filters (7), an amplitude detector (8), a narrowband low-frequency amplifier (9), and a synchronous detector (10), an integrator (11), a direct current amplifier (12).

At the receiver outlet, voltage is formed that is proportional to the difference of the noise temperature Ta from the aerial outlet and the noise temperature Tra from the attenuator outlet:

$$\Delta U = k \left( T_a - T_{ra} \right),$$

where

k is the amplification coefficient of the radiometer receive path,
Ta is the noise temperature from the aerial outlet,
Tra is the noise temperature from the attenuator outlet.

This voltage is amplified and comes onto the Peltier element (16). Same as in the first option of the invention embodiment shown on Fig.3, 2 loads are installed on the Peltier element, which have a good thermal contact with the Peltier element. However, the first load (6) is connected to the inlet of attenuator (18), which is connected to the selector, while the second load is connected to the third shoulder of circulator (4).

The temperature of loads is measured with the help of a temperature sensor (7), which can be installed on the Peltier element and/or at least on one of the loads and has a good thermal contact with them.

Due to negative feedback, voltage at the synchronous detector outlet tends toward zero while noise temperature $Tr_1$ of the first load comes close to the noise temperature Ta from the aerial outlet. Due to dissipation losses in the attenuator (18), the temperature of the first and second loads differs from the temperature Ta of noise coming from the aerial outlet.

$$Tr1 = Tr2 = \frac{Ta}{kra} - (1 - kra)\frac{Tamb}{kra},$$

where

$k_{ra}$ is the transmission coefficient of the attenuator,
$Tr_1$ is the noise temperature of the first load,
$Tr_2$ is the noise temperature of the second load,
Ta is the noise temperature from the aerial outlet,
T amb is the noise temperature of the inlet part of the radiometer.

The power of noise coming on the aerial inlet on the side of the second load (17) is equal to:

$$Tra = Tr2 * k_s k_{cir} + (1 - k_s k_{cir}) * Tamb,$$

where

$k_s$ is the transmission coefficient of the selector,
$k_{cir}$ is the transmission coefficient of the circulator,
Tra is the noise temperature of the attenuator,
$Tr_2$ is the noise temperature of the second load,
Tamb is the noise temperature of the inlet part of the radiometer.

If the transmission coefficient k of the attenuator coincides with the transmission coefficient of the cascade connection of the circulator and selector $k_s k_{cir}$, then

$$Tra = Ta,$$

that is compensation of the noise reflected from the aerial inlet occurs.

So, in the design of radiometer according to the claimed invention, in the modulator a selector is used instead of a switch, and two microwave loads. Wherein, the first microwave load can be connected to the selector, the second microwave load is connected to the third shoulder of the circulator, and the selector is made with the faculty of connecting to the first shoulder of the circulator either the aerial applicator or the first microwave load. Besides, between the outlet of the first microwave load and the selector, the attenuator (18) is preferably installed, and in such case, the selector connects to the first shoulder of the circulator either the aerial (1) or the attenuator.

Such modification of the design of radiometer according to the claimed invention provides a higher accuracy of the non-invasive measurement of temperature of the inner tissues of biological objects with use for early diagnosis of oncological diseases, also provides reduced dimensions of the instrument, improved convenience of its use, and lower cost of its manufacture.

**Claims**

1. A Radiometer containing, sequentially connected, an aerial (1) applicator contacting a biological object, a selector (2"),
a circulator (3, 4) installed after the selector (2"),
a receiver (5) containing an amplifier (9) with bandpass filters (7), an amplitude detector (8), a narrowband low-frequency amplifier (9) and an synchronous detector (10), an integrator (11), a direct current amplifier (12), a reference voltage generator (3) connected to the selector (2") and synchronous detector (10),
a Peltier element (16) connected to the receiver (5) inlet,
the first and second microwave loads (6) installed on the Peltier element (16) and having thermal contact with it,
at least one temperature sensor (13, 7) made with the faculty of measuring the temperature of microwave loads (6), wherein
the first microwave load (6) is made with the faculty of connection to the selector (2"),
the selector (2") is made with the faculty of connecting to the first shoulder of the circulator (3, 4) either the aerial (1) applicator or the first microwave load (6),
the second shoulder of the circulator (3, 4) is connected to the receiver (5), and
the third shoulder of the circulator (3, 4) is connected to the second microwave load (6).

2. The radiometer according to claim 1 containing additionally an attenuator : (18) installed between the outlet of the first microwave load (6) and the selector (2").

3. The radiometer according to claim 1, in which at least one temperature (13, 7) sensor is installed on the Peltier (16) element and/or on the microwave loads (6).

4. The radiometer according to claim 1, in which at least one temperature sensor (13, 7) is an infrared sensor made with the faculty of remote temperature measurement.

5. The radiometer according to any of claims 1-3 containing an additional integrator (14) connected to the outlet of at least one temperature sensor (13, 7).

**Patentansprüche**

1. Radiometer, enthaltend, sequentiell verbunden bzw. geschaltet,
einen Antennen(1)-Applikator, der ein biologisches Objekt berührt,
einen Selektor (2"),
einen Zirkulator (3, 4), der nach dem Selektor (2") installiert ist,
einen Empfänger (5) mit einem Verstärker (9) mit Bandpassfiltern (7), einem Amplitudendetektor (8), einem Schmalband-Niederfrequenzverstärker (9) und einem Synchrondetektor (10), einem Integrator (11), einem Gleichstromverstärker (12), einem Referenzspannungsgenerator (3), der mit dem Selektor (2") und dem Synchrondetektor (10) verbunden ist, ein Peltier-Element (16), das mit dem Empfänger(5)-Eingang verbunden ist, wobei die erste und die zweite Mikrowellenlast (6) auf dem Peltier-Element (16) installiert sind und mit diesem thermischen Kontakt haben,
zumindest einen Temperatursensor (13, 7), gemacht mit der Fähigkeit, die Temperatur von Mikrowellenlasten (6) zu messen, wobei
die erste Mikrowellenlast (6) mit der Fähigkeit der Verbindung mit dem Selektor (2") gemacht ist,
der Selektor (2") mit der Fähigkeit gemacht ist, mit der ersten Schulter des Zirkulators (3, 4) entweder den Antennen(1)-Applikator oder die erste Mikrowellenlast (6) zu verbinden,
die zweite Schulter des Zirkulators (3, 4) mit dem Empfänger (5) verbunden ist, und
die dritte Schulter des Zirkulators (3, 4) ist mit der zweiten Mikrowellenlast (6) verbunden ist.

2. Radiometer nach Anspruch 1, das zusätzlich ein Dämpfungsglied (18) enthält, das zwischen dem Auslass der ersten Mikrowellenlast (6) und dem Selektor (2") installiert ist.

3. Radiometer nach Anspruch 1, wobei zumindest ein Temperatursensor (13, 7) an dem Peltier-Element (16) und/oder an den Mikrowellenlasten (6) installiert ist.

4. Radiometer nach Anspruch 1, wobei zumindest ein Temperatursensor (13, 7) ein Infrarotsensor ist, der mit der Fähigkeit der Ferntemperaturmessung gemacht ist.

5. Radiometer nach einem der Ansprüche 1-3, das einen zusätzlichen Integrator (14) enthält, der mit dem Auslass mindestens eines Temperatursensors (13, 7) verbunden ist.

**Revendications**

1. Radio-thermomètre contenant, connectés de manière séquentielle,
un applicateur-antenne (1) entrant en contact avec un objet biologique, un sélecteur (2"),
un circulateur (3, 4) installé après le sélecteur (2"),
un récepteur (5) contenant un amplificateur (9) avec des filtres passe-bande (7), un détecteur d'amplitude (8), un amplificateur basse-fréquence à bande étroite (9) et un détecteur synchrone (10), un intégrateur (11), un amplificateur de courant continu (12), un générateur de tension de référence (3) connecté au sélecteur (2") et au détecteur synchrone (10), un élément Peltier (16) connecté à l'entrée du récepteur (5),
les première et seconde charges hyperfréquences (6) installées sur l'élément Peltier (16) et en contact thermique avec celui-ci,
au moins un capteur de température (13, 7) conçu pour pouvoir mesurer la température des charges hyperfréquences (6), dans lequel la première charge hyperfréquence (6) est conçue pour pouvoir se connecter au sélecteur (2"),

le sélecteur (2") est conçu pour pouvoir connecter au premier épaulement du circulateur (3, 4) soit l'applicateur-antenne (1) soit la première charge hyperfréquence (6),

le deuxième épaulement du circulateur (3, 4) est connecté au récepteur (5), et

le troisième épaulement du circulateur (3, 4) est connecté à la seconde charge hyperfréquence (6).

2. Radio-thermomètre selon la revendication 1, contenant en outre un atténuateur (18) installé entre la sortie de la première charge hyperfréquence (6) et le sélecteur (2").

3. Radio-thermomètre selon la revendication 1, dans lequel au moins un capteur de température (13, 7) est installé sur l'élément Peltier (16) et/ou sur les charges hyperfréquences (6).

4. Radio-thermomètre selon la revendication 1, dans lequel au moins un capteur de température (13, 7) est un capteur infrarouge conçu pour pouvoir mesurer la température à distance.

5. Radio-thermomètre selon l'une quelconque des revendications 1 à 3, contenant un intégrateur supplémentaire (14) connecté à la sortie d'au moins un capteur de température (13, 7).

PRIOR ART

# FIG.1

PRIOR ART

# FIG.2

FIG.3

FIG.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- RU 2082118
- US 4235107 A, Ludeke
- RU 2485462
- WO 2485462 A

### Non-patent literature cited in the description

- **VAISBLAT A.V.** Medical Radiometer RTM-01-RES. *Biomedical Technologies and Radio Electronics,* 2001, 11-23
- **VAISBLAT A.V.** Medial Radiometer RTM-01-RES. *Biomedical Technologies and Radio Electronics,* 2001, 11-23